# EUROPEAN PATENT SPECIFICATION

(11) **EP 2 685 899 B1**
(45) Date of publication and mention of the grant of the patent: **20.05.2015**
(21) Application number: 12713340.3
(22) Date of filing: 13.03.2012
(51) Int. Cl.: A61B 6/03

(54) **MULTIPLE MODALITY CARDIAC IMAGING**
MULTIMODALE HERZBILDGEBUNG
IMAGERIE CARDIAQUE MULTIMODALITÉ

(30) Priority: 17.03.2011 US 201161453565 P
(43) Date of publication of application: 22.01.2014
(73) Proprietor: Koninklijke Philips N.V., 5656 AE Eindhoven (NL)
(72) Inventor: HANSIS, Eberhard Sebastian, NL-5656 AE Eindhoven (NL)
(74) Representative: Steffen, Thomas
(86) International application number: PCT/IB2012/051183
(87) International publication number: WO 2012/123896

(56) References cited:
- WO-A1-2010/084389
- WO-A2-2009/060344
- US-A1- 2007 131 858
- US-A1- 2008 107 229
- US-A1- 2009 110 256

## Description

The present application relates to medical imaging arts. It finds particular application in coronary artery imaging and perfusion studies using multiple modality imaging systems which perform both nuclear imaging and x-ray/CT imaging.

In diagnostic nuclear imaging, a radionuclide distribution is studied as it passes through a patient's bloodstream for imaging the circulatory system or for imaging specific organs that accumulate the injected radiopharmaceutical. Advantageously, the radiopharmaceutical can be designed to concentrate in selected tissues to provide preferential imaging of those selected tissues.

In single-photon emission computed tomography (SPECT), one or more radiation detectors, commonly called gamma cameras, are used to detect the radiopharmaceutical via radiation emission caused by radioactive decay events. Typically, each gamma camera includes a radiation detector array and a collimator disposed in front of the radiation detector array. The collimator defines a linear or small-angle conical line of sight so that the detected radiation represents projection data. If the gamma cameras are moved over a range of angular views, for example over a 180° or 360° angular range, then the resulting projection data can be readily reconstructed into an image of the radiopharmaceutical distribution in the patient.

In positron emission tomography (PET), the radioactive decay events of the radiopharmaceutical produce positrons. Each positron interacts with an electron to produce a positron-electron annihilation event that emits two 180° oppositely directed gamma rays. Using coincidence detection circuitry, a ring array of radiation detectors surrounding the imaging patient detect the coincident oppositely directed gamma ray events corresponding to the positron-electron annihilation. A line of response (LOR) connecting the two coincident detections contains the position of the positron-electron annihilation event. Such lines of response can be reconstructed to produce an image of the radiopharmaceutical distribution.

In time-of-flight PET (TOF-PET), the small time difference between the detection times of the two coincident y ray events is used to localize the annihilation event along the LOR (line of response).

In computed tomography (CT) imaging, a radiation source irradiates an imaging subject; and a radiation detector array disposed on the opposite side of the imaging subject detects the transmitted radiation. Due to varying attenuations of radiation by tissues in the imaging subject, the detected radiation can be reconstructed into an image depicting radiation-absorbing structures in the imaging subject.

Diagnosis and treatment planning for coronary artery disease can be performed using different imaging modalities. The most common imaging procedures include myocardial perfusion with nuclear imaging systems, namely SPECT though PET can also be used, and planar x-ray coronary angiography are used to detect coronary artery stenosis and corresponding perfusion defects. While planar 2D angiography is currently preferred in clinical settings due to cost and availability, 3D CT coronary artery imaging is gaining popularity because it offers more information, is potentially easier to interpret than a series of 2D projection images from different angles, and provides quantitative analysis of vessel properties useful in intervention and planning.

The more common catheter-based x-ray coronary angiography is generally performed on a C-arm x-ray system. The C-arm system's flat-panel x-ray detector delivers high-resolution planar 2D angiograms. 3D coronary artery imaging is also possible on the C-arm x-ray system by using a rotational angiography (3DRA) acquisition in conjunction with specially adapted reconstruction algorithms. As an alternative, 3D coronary artery imaging can be performed with a standard x-ray CT system. However, these examinations cannot provide the same functional information as a SPECT scan.

Combining cardiac SPECT and x-ray/CT angiography imaging can improve the diagnosis of cardiac disease by combining functional and anatomic information about the health of the heart and, in particular, of the coronary arteries. Typically, SPECT and x-ray imaging procedures are carried out on separate imaging systems and therefore at different times and locations. It is advantageous to perform both procedures on the same imaging device, to increase patient throughput, reduce risk of mis-registrations or anatomical changes between exams, and increase patient comfort by shortening the total examination time.

Most currently available multiple modality SPECT/CT imaging devices employ a standard CT with a rotating gantry. A standard CT, however, delivers inherently lower imaging resolution than a flat-panel based system and does not provide planar angiograms. Also, co-planar SPECT and CT imaging is not possible when using a standard SPECT/CT system, because the two imaging systems are mounted on two separate rotation gantries.

WO2009/060344 A2 discloses a method according to the preamble of claim 1.

Flat-panel based multiple modality SPECT/CT imaging systems have been proposed with the x-ray detector centered relative to the x-ray source. These systems have been described for use in ECG-gated SPECT and CT studies and can provide high spatial resolution for coronary artery imaging. However, currently available flat-panel detectors are not large enough to cover the whole width of typical patients, when used in a geometry with the detector centered relative to the x-ray source. Therefore, the acquired CT image will likely suffer from truncation because the patient outline is unknown. Therefore, an accurate, non-truncated attenuation correction map cannot be generated from these systems to be used in SPECT reconstruction.

The present application provides a new and improved multiple modality imaging system and method which overcomes the above-referenced problems and others.

In accordance with one aspect, a method for diagnostic imaging is presented. The method includes receiving contrast enhanced CT projection data acquired from an examination region with a laterally offset flat panel detector. A field-of-view (FOV) which includes one or more contrast enhanced vessels is selected. From the received CT projection data, a three-dimensional (3D) vessel image representation, at least one planar vessel angiogram, and a 3D attenuation correction (AC) map of the selected FOV are generated. The step of generating the 3D attenuation correction map includes: segmenting the contrast enhanced vessels in the 3D volume representation, replacing the segmented contrast enhanced vessels with background intensity data, and generating the 3D attenuation correction map based on the CT projection data in which the contrast enhanced vessels are subtracted and replaced with background intensity data. Further, nuclear projection data acquired from the examination region are received. The acquired nuclear projection data are then corrected based on the generated 3D attenuation correction map, and finally a nuclear image representation of the selected FOV is generated from the acquired nuclear projection data based on the corrected nuclear projection data.

In accordance with another aspect, a diagnostic imaging system is presented. The system comprises an x-ray scanner which acquires contrast enhanced CT projection data from an examination region with a laterally offset flat panel detector. The system includes a graphical user interface for selecting a field-of-view (FOV) which includes one or more contrast enhanced vessels. A CT reconstruction processor generates a three-dimensional (3D) vessel image representation, at least one planar vessel angiogram, and a 3D attenuation correction (AC) map of the selected FOV from the acquired CT projection data, wherein the CT reconstruction processor is further configured to segment the contrast enhanced vessels in the 3D volume representation, to replace the segmented contrast enhanced vessels with background intensity data, and to generate the 3D attenuation correction map based on the CT projection data in which the contrast enhanced vessels were subtracted and replaced with background intensity data. The system further includes a nuclear imaging scanner which acquires nuclear projection data from the examination region. A nuclear reconstruction processor is programmed to correct the acquired nuclear projection data based on the generated attenuation correction map, and to generate a nuclear image representation of the selected FOV from the acquired nuclear projection data based on the corrected nuclear projection data.

One advantage is that diagnosis of coronary artery disease is improved. Another advantage resides in reduced examination costs and total examination times.

Another advantage is that the risk of mis-registration is reduced.

Another advantage is that the risk of reduced data quality caused by patient motion and/or anatomical changes between examinations is reduced.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 is diagrammatic view of combined SPECT/CT single gantry system with an offset flat-panel detector;
FIGURE 2 is an example of a 3D coronary artery reconstruction;
FIGURE 3A is an example of a 2D "half" projection, while FIGURE 3B is an example of a corresponding 2D "fused" projection;
FIGURES 4A and 4B are an example of a 2D angiogram and the corresponding angiogram after removal of contrast enhanced vessels; and
FIGURES 5 and 6A-6C are flowcharts of a method for multiple modality diagnostic imaging.

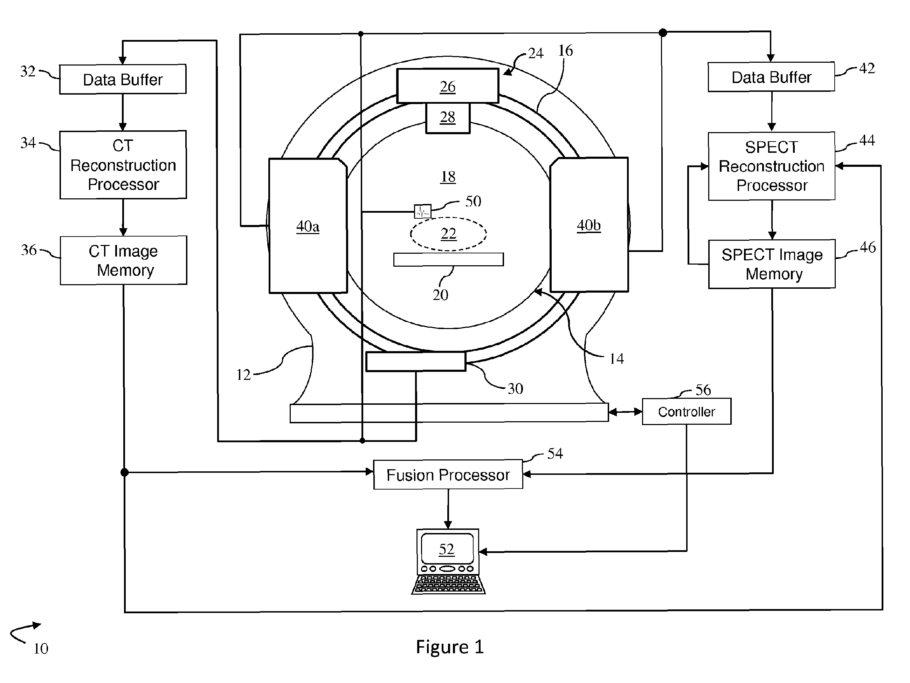

A multiple modality diagnostic imaging system, capable of x-ray CT and nuclear imaging acquisitions, enables comprehensive assessment of coronary artery disease with a single x-ray CT acquisition. During a single imaging session, a cardiac perfusion SPECT reconstruction, 2D x-ray angiograms, and a 3D x-ray coronary artery reconstruction are generated. The system improves diagnosis of coronary artery disease by generating more comprehensive examinations which are accessible to more patients, reducing examination costs and total examination times, and by reducing risk of mis-registration and mis-diagnosis caused by patient motion and anatomical changes between imaging exams. The system is not limited to cardiac imaging studies and can be applied to study various vasculature studies through a patient's body, such as neural. With reference to FIG. 1, a diagnostic imaging system **10** performs concurrently and/or independently x-ray computed tomography (CT) and nuclear imaging, such as PET or SPECT. The imaging system **10** includes a stationary housing **12** which defines a patient receiving bore **14.** A rotatable gantry **16,** supported by the housing **12,** is arranged for rotation around the bore to define a common examination region **18.** A patient support **20,** which supports a patient or subject **22** to be imaged and/or examined, is longitudinally and/or vertically adjusted to achieve the desired positioning of the patient in the examination region.

To provide CT imaging capabilities, an x-ray assembly **24** which is mounted on the rotatable gantry **16** includes an x-ray source **26,** such as an x-ray tube, and a collimator or shutter assembly **28** which, in addition to the collimator, may include various filters to modify the spectral characteristics of the emitted x-ray radiation. The collimator collimates the radiation from the x-ray source **26** into a cone or wedge beam, one or more substantially parallel fan beams, or the like. The shutter gates the beam on and off. An x-ray detector **30,** such as a solid state, flat panel detector, is mounted on the rotatable gantry **16** opposite the x-ray assembly **24.** In the illustrated embodiment, the detector panel is laterally offset relative to the projected center of radiation or transversely displaced from the center of rotation in the trans-axial plane. More specifically, the cone beam and the CT detector **30** are offset such that slightly more than half of the field-of-view (FoV) is examined in each single x-ray projection. The whole FoV can be examined when the x-ray source and the detector rotate approximately 360°. Offset detector geometries are desirable because they allow for an increased FoV given a fixed detector size or allow for smaller detectors sizes. Larger detectors tend to be more complex, expensive to manufacture, can limit the overall system design, and can limit detector positioning or patient access or the like.

As the gantry rotates, the x-ray assembly **24** and the x-ray detector **30** revolve in concert around the examination region **18** to acquire CT projection data spanning a full 360° revolution, multiple revolutions, or a smaller arc. Each CT projection indicates x-ray attenuation along a linear path between the x-ray assembly **24** and a detecting element of the x-ray detector **30.** The acquired CT projection data is stored in a CT data buffer **32** and processed by a CT reconstruction processor **34** into a CT image representation and then stored in a CT image memory unit **36.** Taken together, the x-ray source, the collimator/shutter assembly, the detector, and the reconstruction processor define a system or means for generating an anatomical, CT, x-ray, or first image.

To provide nuclear imaging capabilities, at least two nuclear detector heads **40a, 40b,** such as single photon emission tomography (SPECT) detectors, are moveably mounted to the rotating gantry **16.** Mounting the x-ray assembly **24** and the nuclear detector heads **40a, 40b** on the same rotatable gantry permits the examination region **18** to be imaged by both modalities without moving the patient **22**,i.e. co-planar imaging. In one embodiment, the detector heads are moveably supported by a robotic assembly (not shown) which is mounted to the rotating gantry **16.** The robotic assembly enables the detector heads to be positioned at a selectable offset about the patient **22,** e.g. 90° offset, 180° opposite each other, etc. Each SPECT detector head includes a collimator such that each detected radiation event is known to have originated along an identifiable linear or small-angle conical line of sight so that the acquired radiation comprises projection data. The acquired SPECT projection data is stored in a data buffer **42** and processed by a SPECT reconstruction processor **44** into a SPECT image representation and stored in a SPECT image memory unit **46.** Taken together, the SPECT detector heads and the SPECT reconstruction processor define a system or means for generating a nuclear, functional, or second image.

In another embodiment, the nuclear imaging system or means includes positron emission tomography (PET) detectors, not illustrated, rather than the SPECT detectors **40a, 40b.** One or more rings of PET detectors are arranged about the patient receiving bore **14** to receive gamma radiation therefrom. Detected pairs of coincident radiation events define LORs which are stored in list mode in a data buffer and processed by a PET reconstruction processor into a PET image representation and stored in a PET image memory unit. Taken together, the PET detector ring(s) and the PET reconstruction processor define the system or means for generating the functional image. It should be appreciated that the combination of a flat panel x-ray assembly 24 and a PET system in a single gantry is not illustrated but is also contemplated.

Combining cardiac SPECT and CT angiography imaging can improve diagnosis of cardiac disease by combining functional and anatomical information about the health of the heart and the coronary arteries. The imagining system **10** can acquire co-planar SPECT and CT images along with non-truncated attenuation correction (AC) maps which are then reconstructed and presented to a clinician as fused and non-fused views on a display.

For a cardiac imaging procedure, a patient **22** is positioned on the support **20.** An electrocardiogram (ECG) recording device **50** is positioned on or near the patient to record the patients ECG signal during CT and SPECT data acquisition to gate the acquired projection data. It should also be noted that the ECG signal can be replaced by suitable methods that derive the subject's heart phase based on the acquired image data, e.g. CT or nuclear image data, without using the additional EGC recording device **50.** An x-ray contrast enhancing agent is administered to the patient to enhance the contrast of the coronary arteries during acquisition. The contrast agent is injected intravenously or via a catheter directly into the coronary arteries such that a constant concentration of the contrast agent is present in the coronary arteries throughout the CT acquisition.

A 360-degree x-ray acquisition is performed using the CT component of the system **10** which consists of the x-ray source **26** and the laterally offset flat-panel x-ray detector **30,** mounted on the same gantry as the SPECT detector heads **40a, 40b.** As previously mentioned, the detector offset enables imaging over the entire patient axial cross-section. Each X-ray projection covers slightly more than half of the patient. Using the ECG recording device **50,** the patient's ECG signal is recorded during the CT acquisition and is temporally registered to the x-ray acquisition. The acquired CT projection data and corresponding ECG data are stored in the CT data buffer **32.**

In one embodiment, a 3D reconstruction of the coronary arteries is generated from the stored CT projection data that represent a selected cardiac motion state. In one embodiment, a clinician selects a cardiac motion state for reconstruction, e.g. in the late diastole where heart motion is relatively small. In another embodiment, the CT reconstruction processor **34** automatically selects the optimum cardiac motion state. The corresponding projections for reconstruction are determined according to the ECG gating signal and the selected optimum cardiac motion state. The CT reconstruction processor **34** is programmed to pre-filter the gated CT projection data to enhance the coronary arteries and reduce the anatomic background. Filtering methods include morphological filtering such as a 'top-hat' filter, multi-scale vesselness filtering with vessel segmentation, a combination of these two methods, or other known filtering methods. The background removal is beneficial in order to achieve high-quality coronary artery iterative reconstruction from few projections, which will be later described, to enable reconstruction of high-resolution sub-volume image representation containing only the coronary arteries.

Because the ECG gating results in a limited number of projections available for each cardiac motion state, the reconstruction processor **34** performs a Few-Projections reconstruction algorithm. The reconstruction algorithm is an iterative reconstruction method which uses at least one of the spatial sparseness of the coronary arteries as a regularization factor, a redundancy weighting factor to account for the central overlap region, and a small update step to achieve uniform convergence with the truncated projection data and non-uniform volume coverage. The reconstruction can be performed in a small, high-resolution sub-volume containing the coronary arteries only. Also, several motion states can be reconstructed to generate an image in each motion state. The reconstruction can incorporate motion estimation and motion correction steps, e.g., projection-based motion correction or volume-based motion estimation and correction, to correct for cardiac motion or other residual motion. For example, a motion model can be derived which models the motion between motion states. The motion model can be used to map or transform images or the underlying projection data from other motion states into the selected motion state. The reconstructed 3D coronary image representation is stored to the CT image memory **36.**

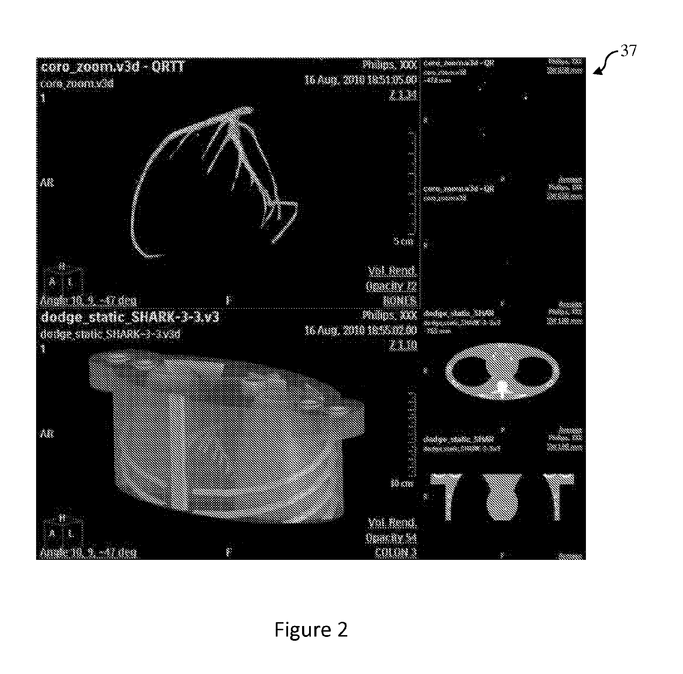

FIGURE 2 displays an example of an ECG-gated coronary artery reconstruction **37** in a software phantom study for a simulated offset-detector flat-panel X-ray acquisition. The 3D high-resolution reconstruction of the coronary arteries was generated from 18 projections that were equi-angularly spaced over 360°. The projection selection corresponds to a nearest-neighbor ECG gating on a 12 second scan time at a heart rate of 90 bpm. The reconstruction was generated from top-hat filtered projections using the iterative reconstruction method which uses the sparseness regularization, redundancy weighting factor, and a small update step.

In another embodiment, at least one 2D angiogram is generated from the acquired CT projection data of the selected cardiac motion state. The selected projection data can be filtered to enhance the visibility of the coronary arteries using known filter methods, such as contrast enhancement, histogram optimization, vesselness filtering and vessel segmentation, or morphological filters.

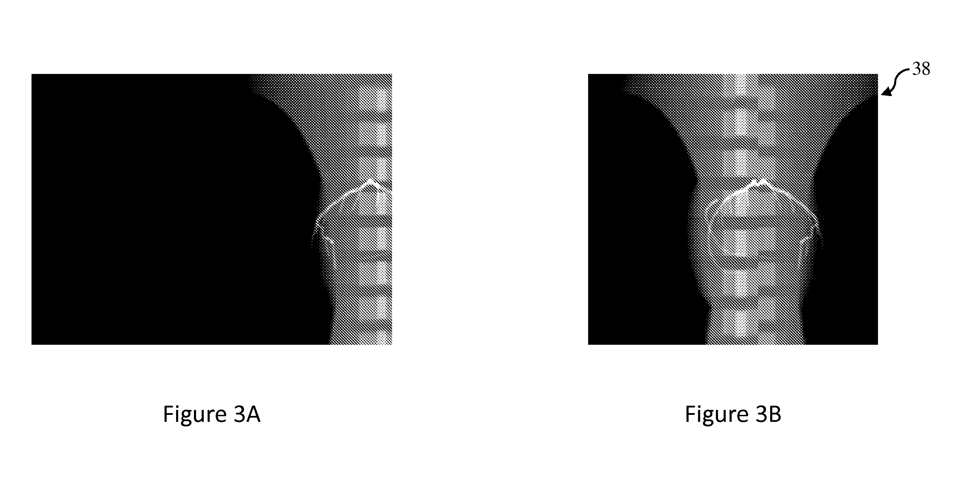

For a given gantry rotation angle, the CT reconstruction processor **34** selects the corresponding x-ray projection stored in the buffer **32.** The selected projection shows the coronary artery tree truncated because the offset-detector geometry captures a little over half of the FOV. A second projection acquired in the same cardiac motion state as the first is selected from the buffer **32** such that the difference between the projection angles of the first and second projection is as close to 180° degrees as possible. The CT reconstruction processor **34** fuses the two projections resulting in one 2-D angiogram for the two given projection angles and stores the composite 2D angiograms to the CT image memory **36.** Figures 3A and 3B display an example of a first 2D truncated angiogram and the fused 2-D angiogram **38,** respectively.

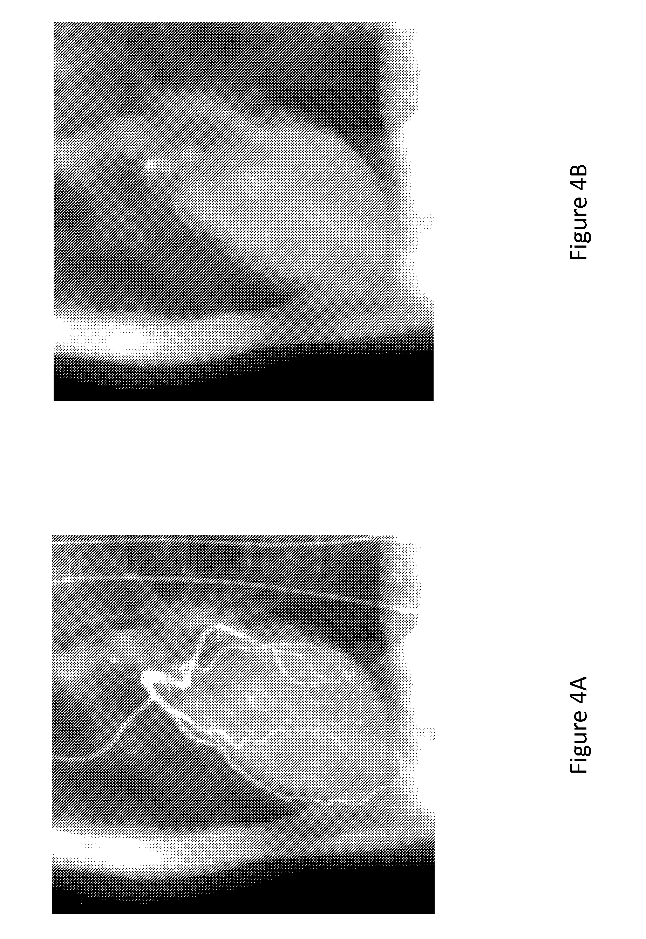

In an embodiment not covered by the prsent invention, an attenuation correction (AC) map is generated from the acquired CT projection data. The presence of the contrast enhancing agent in the CT projections scan can interfere in the generation of an accurate AC map because the contrast agent is not present during the SPECT acquisition. The AC map with the contrast enhancing agent present in the coronary arteries can produce inconsistent attenuation information for SPECT reconstruction correction. To remove the contrast enhancement, the CT reconstruction processor **34** segments the contrast enhanced vessels in the projection data using known methods and replaces the segmented regions with a background intensity. According to the present invention, the CT reconstruction processor **34** segments and replaces the contrast enhanced vessels in the reconstructed 3D image data rather than the projection data, i.e. after the projection data is reconstructed into a 3D volume representation. Furthermore, the CT reconstruction processor **34** can segment the contrast enhanced vessels in the 3D volume representation; a forward projection of the segmented vessels can then be subtracted from the projection data. After the contrast enhanced vessels are removed and replaced with background intensity, a 3D reconstruction is performed using known methods for cone-beam CT reconstruction with a laterally offset-detector flat-panel system, such as a filtered backprojection or an iterative reconstruction method. The generated 3D volume can be post-processed for noise removal, truncation correction, contrast medium reduction, or resolution adaptation like down-sampling. The AC map is generated from this 3D reconstruction volume. FIGURE 4A is an example of a 2D angiogram from a C-arm rotational angiography acquisition and FIGURE 4B is the angiogram after removal of contrast-enhanced vessels. In this example, the contrast enhanced vessels were detected and segmented using a multi-scale vesselness filter, subtracted, and replaced with interpolated projection background.

Thus far, the acquired CT projection data yields a 3D coronary artery reconstruction, a series of 2D angiograms, and an attenuation correction map that can be used to correct analogous SPECT projection data. The SPECT projection data can be acquired before, after, or interleaved with the CT data acquisition. To acquire the SPECT data, the patient, which remains stationary in the examination region **18,** is administered a radiopharmaceutical tracer. The SPECT projection data and an ECG gating signal are acquired concurrently and stored on the SPECT image memory **46.** The SPECT reconstruction processor corrects the SPECT projection data based on the AC map and generates a 3D image reconstruction from the corrected SPECT data according to the selected cardiac phase.

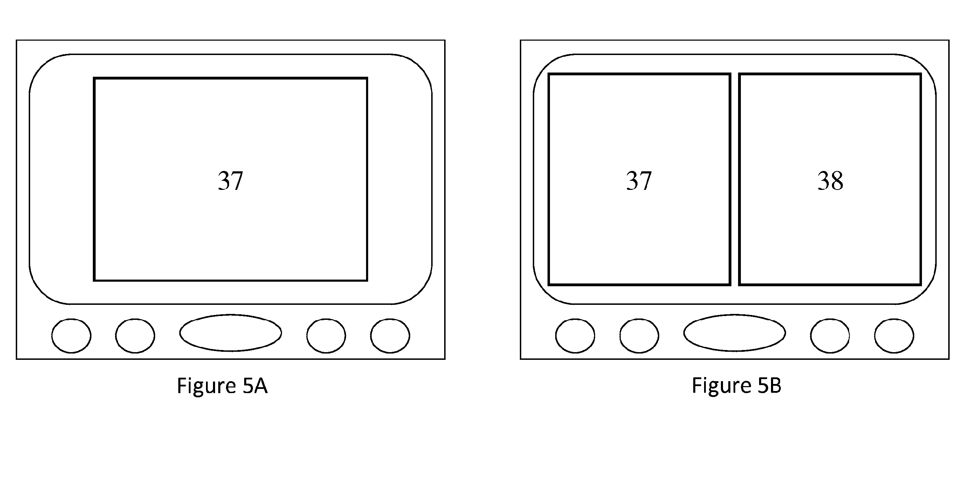

The image representations, e.g. the reconstructed 3D vessel representation, 2D angiograms, and corrected SPECT reconstructions, are visualized by a clinician on a graphical user interface (GUI) **52.** The GUI includes also includes a user input device by which the clinician or user interacts with the system **10**.In one embodiment, shown in FIGURE 5A, the clinician can instruct the GUI 52 to display one of the image representation at a higher resolution to encompass the entire display of the GUI **52.** For example, the clinician instructs the GUI to display the reconstructed 3D coronary artery tree. The clinician can use the user input device to rotate the 3D coronary artery tree to visualize the arteries at various arteries. If the clinician discovers an anomaly, they can instruct the GUI **52** to display the 2D angiogram which corresponds to the rotation angle of the current view. In another embodiment, shown in FIGURE 5B, more than one image representation can be visualized concurrently. Continuing with the same example, the clinician can instruct the GUI **52** to display the corresponding 2D angiogram beside the 3D coronary artery reconstruction. Furthermore, the clinician can display the corrected 3D SPECT reconstruction concurrently with the 3D coronary artery reconstruction at the corresponding rotation angle and/or with a corresponding 2D angiogram. In another embodiment, the image representations are displayed in various superimpositions. The imaging system 10 includes a fusion processor **54** which is programmed with known methods for image registration and fusion of the 2D angiograms, 3D coronary artery reconstruction, and the corrected 3D SPECT reconstruction in various combinations. The graphic user interface **52** also allows the clinician or user to interact with a scan controller **56** to select scanning sequences and protocols, and the like.

With reference to FIGURE 5, a method for multiple modality cardiac imaging is presented. After a patient is positioned in the examination region **18** and the corresponding scanning protocols are selected via the GUI **52,** the patient is injected with an x-ray detectable contrast enhancing medium **S100.** A 360° x-ray acquisition is performed **S110** by rotating the gantry mounted x-ray source **24** and the laterally offset flat panel x-ray detector **30** about the examination region **18.** An ECG signal of the patient is recorded with an ECG recording device **50** during the x-ray acquisition and is temporally registered to the x-ray projection data. Using the GUI **52,** the clinician can select one or more of the patient's cardiac phases **S120** from which the corresponding projection data will be selected for generating a 3D coronary artery reconstruction **S130,** a series of 2D planar coronary artery angiograms **S140,** and a 3D attenuation correction map **S150.**

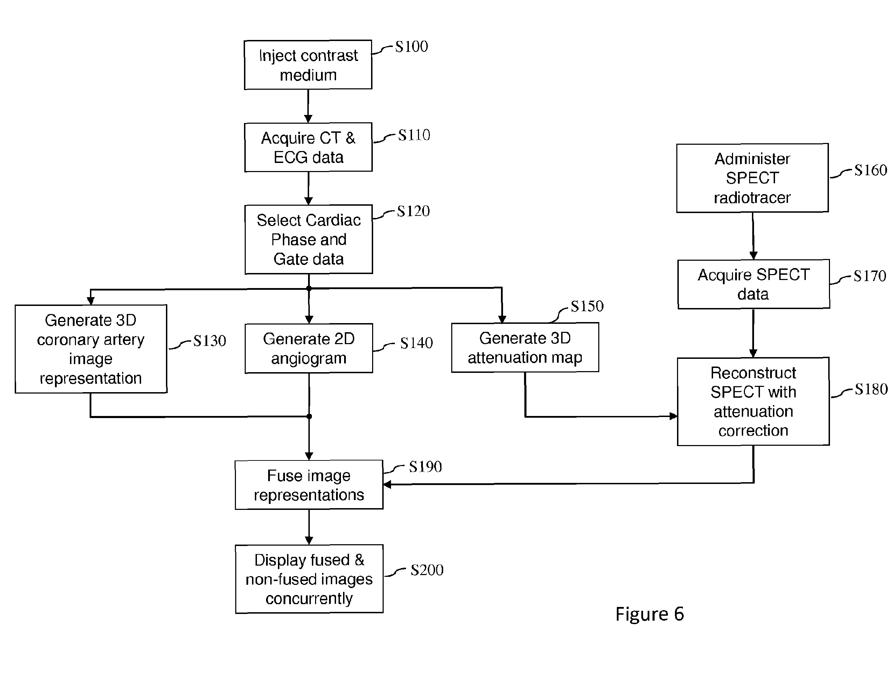

While the patient remains in the examination region **18,** the patient is injected with a SPECT radiotracer **S160** and a cardiac SPECT acquisition is performed **S170** by rotating the SPECT detector heads **40a, 40b,** which are mounted to the same gantry as the x-ray assembly **24, 30,** about the examination region **18.** An ECG signal of the patient is recorded during the SPECT acquisition and then temporally registered to the SPECT projection data. The projection data is reconstructed **S180** into a 3D image representation of the patient's cardiac region using the 3D attenuation correction map and the selected cardiac motion state. The fusion processor **54** combines the 3D x-ray coronary image representation, 2D x-ray planar angiograms, and the 3D SPECT cardiac image representation into various superimpositions which can be beneficial to the clinician diagnosing the cardiac region of the patient. The 3D x-ray coronary image representation, 2D x-ray planar angiograms, the 3D SPECT cardiac image representation, and the various combined image representations are displayed alone or concurrently on a display of the GUI **52.**With reference to FIGURE 6A, the method for the generation of the 3D x-ray coronary artery image representation **S130** is presented. After the cardiac phase is selected and the corresponding projection data is gated **S120,** the projection data is filtered **S210** to enhance the coronary arteries and reduce the anatomic background using at least one of morphological filtering, multi-scale vesselness filtering and vessel segmentation, one or more combinations of these methods, or other known filtering methods. The filtered data is reconstructed **S220** using in iterative reconstruction algorithm adapted for the limited number projection available and the laterally offset detector geometry. The reconstruction can also incorporate motion estimate and compensation to correct for cardiac motion or other residual motion.

With reference to FIGURE 6B, the method for the generation of the 2D planar coronary artery angiograms **S140** is presented. After the cardiac phase is selected and the corresponding projection data is gated **S120,** the projection data is filtered **S240** to enhance the coronary arteries. For each projection angle, the captured field of view is truncated, e.g. coronary artery tree may be truncated, because the offset-detector geometry captures a little over half of the FOV. Therefore, a second projection acquired in the same cardiac motion state as the first is determined **S250** such that the difference between the projection angles of the first and second projection is as close to 180° degrees as possible. The CT reconstruction processor **34** fuses the two projections **S260** resulting in a composite or fused 2D angiogram **S270** for the two given projection angles.

With reference to FIGURE 6C, the method for the generation of the 3D attenuation correction map **S150** is presented. After the cardiac phase is selected and the corresponding projection data is gated **S120,** the CT reconstruction processor **34** removes the contrast enhancement from the projections. It should be noted that the ECG signal may or may not be used in the generation of the AC map. To remove the contrast enhancement, the CT reconstruction processor **34** segments the contrast enhanced vessels **S280** in the projection data, in the 2D planar angiograms generated in step **S140,** or in reconstructed image data using known methods and replaces the segmented regions with a background intensity **S290.** A 3D reconstruction is performed **S300** to generate a 3D volume using a filtered backprojection or iterative algorithm which takes into account the laterally offset flat panel detector geometry. The generated 3D volume is post-processed **S310** for noise removal, truncation correction, contrast medium reduction, or resolution adaptation like down-sampling. AC map is generated **S320** from the processed 3D reconstruction volume.

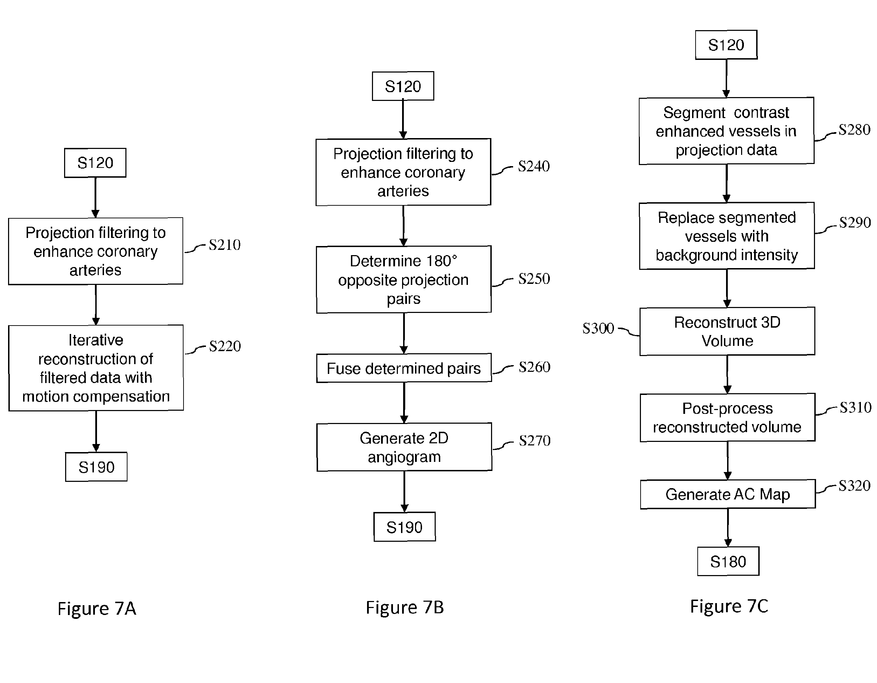

In another embodiment, a computer readable medium having embodied thereon a computer program or instructions for controlling a processor for performing the cardiac imaging method of FIGURES 6 and FIGURES 7A-7C is provided.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

## Claims

1. A method for diagnostic imaging, comprising:
receiving contrast enhanced CT projection data acquired from an examination region (18) with a laterally offset flat panel detector (30);
selecting a field-of-view (FOV) which includes one or more contrast enhanced vessels;
from the received CT projection data, generating a 3D attenuation correction (AC) map of the selected FOV and at least one of a three-dimensional (3D) vessel image representation and at least one planar vessel angiogram;
receiving nuclear projection data acquired from the examination region (18);
correcting the acquired nuclear projection data based on the generated 3D attenuation correction map; and
generating a nuclear image representation of the selected FOV from the acquired nuclear projection data based on the corrected nuclear projection data; **characterised in that** the step of generating the 3D attenuation correction map includes: segmenting the contrast enhanced vessels in the 3D volume representation, replacing the segmented contrast enhanced vessels with background intensity data, and generating the 3D attenuation correction map based on the CT projection data in which the contrast enhanced vessels are subtracted and replaced with background intensity data

2. The method according to claim 1, wherein the step of generating a three-dimensional (3D) vessel image representation, includes:
filtering the received CT projection data to enhance the vessels and to remove background information in the selected FOV; and
reconstructing a 3D image representation of the FOV from the filtered projection data.

3. The method according to claim 2, wherein the step of generating a three-dimensional (3D) vessel image representation, further includes:
correcting at least one of the filtered projection data and the reconstructed 3D image representation for motion.

4. The method according to any one of claims 1-3, wherein the reconstruction is performed with an iterative reconstruction algorithm with at least one of a regularization factor, a redundancy weighting factor, and a small update step.

5. The method according to any one of claims 1-4, wherein the step of generating at least one planar vessel angiogram, includes:
filtering the received projection data to enhance the vessels in the selected FOV;
for each projection angle, generating a first 2D truncated angiogram and a second 2D truncated angiogram, the second 2D truncated angiogram having a projection angle approximately 180° opposite from the given projection angle during a similar heart motion state; and
generating 2D composite angiogram for each projection angle by fusing the first and second 2D truncated angiograms.

6. The method according to claim 1, further including:
combining the nuclear image representation, the 3D vessel image representation, and the at least one planar vessel angiogram into a composite image; and
displaying the nuclear image representation, the 3D vessel image representation, the at least one planar vessel angiogram, and the composite image.

7. The method according to claim 1, further including:
receiving electrocardiogram (ECG) data acquired during the acquisition of the CT projection data and the nuclear projection data; and
prior to generating the image representations and the at least one angiogram, gating the CT and nuclear projection data according to a selected cardiac motion state.

8. A computer-readable medium carrying software for controlling one or more processors to perform the method according to any one of claims 1-7.

9. A system (10) for diagnostic imaging, comprising:
an x-ray scanner (24,30) which acquires contrast enhanced CT projection data from an examination region (18) with a laterally offset flat panel detector (30);
a graphical user interface (50) for selecting a field-of-view (FOV) which includes one or more contrast enhanced vessels;
a CT reconstruction processor (34) which generates a 3D attenuation correction (AC) map of the selected FOV and at least one of a three-dimensional (3D) vessel image representation and at least one planar vessel angiogram, from the acquired CT projection data;
a nuclear imaging scanner (40a,40b) which acquires nuclear projection data from the examination region (18);
a nuclear reconstruction processor (44) programmed to correct the acquired nuclear projection data based on the generated attenuation correction map, and to generate a nuclear image representation of the selected FOV from the acquired nuclear projection data based on the corrected nuclear projection data;
**characterised in that** the CT reconstruction processor (34) is further configured to segment the contrast enhanced vessels in the 3D volume representation, to replace the segmented contrast enhanced vessels with background intensity data, and to generate the 3D attenuation correction map based on the CT projection data in which the contrast enhanced vessels were subtracted and replaced with background intensity data.

10. The diagnostic imaging system (10) according to claim 9, wherein the CT reconstruction processor (34) is programmed to:
filter the acquired CT projection data to enhance the contrast enhanced vessels and to remove background information in the selected FOV; and
reconstruct the 3D vessel image representation of the FOV from the filtered projection data.

11. The diagnostic imaging system (10) according to claim 10, wherein the CT reconstruction processor (34) is further programmed to:
correct at least one of the filtered projection data and the reconstructed 3D vessel image representation for motion.

12. The diagnostic imaging system (10) according to either one of claims 10 and 11, wherein the reconstruction is performed with an iterative reconstruction algorithm with at least one of a regularization factor, a redundancy weighting factor, and a small update step.

13. The diagnostic imaging system (10) according to any one of claims 9-12, wherein the CT reconstruction processor (34) is programmed to:
filter the acquired projection data to enhance the vessels in the selected FOV;
generate a first 2D truncated angiogram and a second 2D truncated angiogram for each projection angle, the second 2D truncated angiogram having a projection angle approximately 180° opposite from the given projection angle during a similar heart motion state; and
generate 2D composite angiogram for each projection angle by fusing the first and second 2D truncated angiograms.

14. The diagnostic imaging system (10) according to claim 9, further including:
a fusion processor (54) which combines the nuclear image representation, the 3D vessel image representation, and the at least one planar vessel angiogram into a composite image; and
a graphical user interface (52) which displays the nuclear image representation, the 3D vessel image representation, the at least one planar vessel angiogram, the composite image, or any combination thereof.

15. The diagnostic imaging system (10) according to claim 9, further including:
an ECG device (50) which acquires electrocardiogram (ECG) data during the acquisition of the CT projection data and the nuclear projection data; and
prior to generating the image representations and the at least one angiogram, gating the CT and nuclear projection data according to a selected cardiac motion state.

## Patentansprüche

1. Verfahren zur diagnostischen Bildgebung, das Folgendes umfasst:
Empfangen von kontrastverstärkten CT-Projektionsdaten, die mit einem seitlich versetzten Flachdetektor (30) aus einer Untersuchungsregion (18) erfasst wurden;
Auswählen eines Sichtfelds (field-of-view, FOV), das ein oder mehrere konstrastverstärkte Gefäße enthält;
Erzeugen einer dreidimensionalen Abschwächungskorrekturkarte (attenuation correction, AC) des ausgewählten Sichtsfelds und mindestens entweder einer dreidimensionalen (3D) Gefäßbilddarstellung oder mindestens eines planaren Gefäßangiogramms anhand der empfangenen CT-Projektionsdaten;
Empfangen von Nuklearprojektionsdaten, die aus der Untersuchungsregion (18) erfasst wurden;
Korrigieren der erfassten Nuklearprojektionsdaten auf der Grundlage der erzeugten dreidimensionalen Abschwächungskorrekturkarte; und
Erzeugen einer Nuklearbilddarstellung des ausgewählten Sichtfelds anhand der erfassten Nuklearprojektionsdaten auf der Grundlage der korrigierten Nuklearprojektionsdaten;
**dadurch gekennzeichnet, dass** der Schritt des Erzeugens der dreidimensionalen Abschwächungskorrekturkarte Folgendes umfasst: Segmentieren der kontrastverstärkten Gefäße in der dreidimensionalen Volumendarstellung, Ersetzen der segmentierten kontrastverstärkten Gefäße durch Hintergrundintensitätsdaten und Erzeugen der dreidimensionalen Abschwächungskorrekturkarte auf der Grundlage der CT-Projektionsdaten, in denen die kontrastverstärkten Gefäße subtrahiert und durch Hintergrundintensitätsdaten ersetzt wurden.

2. Verfahren nach Anspruch 1, wobei der Schritt des Erzeugens einer dreidimensionalen (3D) Gefäßbilddarstellung Folgendes umfasst:
Filtern der empfangenen CT-Projektionsdaten, um in dem ausgewählten Sichtfeld die Gefäße zu verstärken und die Hintergrundinformationen zu entfernen; und
Rekonstruieren einer dreidimensionalen Bilddarstellung des Sichtfelds anhand der gefilterten Projektionsdaten.

3. Verfahren nach Anspruch 2, wobei der Schritt des Erzeugens einer dreidimensionalen (3D) Gefäßbilddarstellung weiterhin Folgendes umfasst:
Korrigieren von mindestens entweder den gefilterten Projektionsdaten oder der rekonstruierten dreidimensionalen Bilddarstellung in Hinblick auf Bewegung.

4. Verfahren nach einem der Ansprüche 1 bis 3, wobei das Rekonstruieren mit einem iterativen Rekonstruktionsalgorithmus mit mindestens entweder einem Regelungsfaktor, einem Redundanzgewichtungsfaktor oder einem kleinen Aktualisierungsschritt durchgeführt wird.

5. Verfahren nach einem der Ansprüche 1 bis 4, wobei der Schritt des Erzeugens von mindestens einem planaren Gefäßangiogramm Folgendes umfasst:
Filtern der empfangenen Projektionsdaten, um die Gefäße in dem ausgewählten Sichtfeld zu verstärken;
Erzeugen eines ersten zweidimensionalen trunkierten Angiogramms und eines zweiten zweidimensionalen trunkierten Angiogramms für jeden Projektionswinkel, wobei das zweite zweidimensionale trunkierte Angiogramm einen Projektionswinkel von ungefähr 180° gegenüber dem gegebenen Projektionswinkel während eines ähnlichen Herzbewegungszustands hat; und
Erzeugen eines zweidimensionalen zusammengesetzten Angiogramms für jeden Projektionswinkel durch Verschmelzen des ersten und des zweiten zweidimensionalen trunkierten Angiogramms.

6. Verfahren nach Anspruch 1, das weiterhin Folgendes umfasst:
Kombinieren der Nuklearbilddarstellung, der dreidimensionalen Gefäßbilddarstellung und des mindestens einen planaren Gefäßangiogramms zu einem zusammengesetztem Bild; und
Anzeigen der Nuklearbilddarstellung, der dreidimensionalen Gefäßbilddarstellung, des mindestens eine planaren Gefäßangiogramms und des zusammengesetzten Bilds.

7. Verfahren nach Anspruch 1, das weiterhin Folgendes umfasst:
Empfangen von während der Erfassung der CT-Projektionsdaten und der Nuklearprojektionsdaten erfassten Elektrokardiogrammdaten (ECG); und
Gating der CT- und Nuklearprojektionsdaten entsprechend einem ausgewählten Herzbewegungszustand, bevor die Bilddarstellungen und das mindestens eine Angiogramm erzeugt werden.

8. Computerlesbares Medium mit Software zur Steuerung von einem oder mehreren Prozessoren, um das Verfahren nach einem der Ansprüche 1 bis 7 durchzuführen.

9. System (10) zur diagnostischen Bildgebung, das Folgendes umfasst:
einen Röntgenscanner (24, 30), der mit einem seitlich versetzten Flachdetektor (30) kontrastverstärkte CT-Projektionsdaten aus einer Untersuchungsregion (18) erfasst;
eine graphische Benutzeroberfläche (50) zum Auswählen eines Sichtfelds (field-of-view, FOV), das ein oder mehrere konstrastverstärkte Gefäße enthält;
einen CT-Rekonstruktionsprozessor (34), der eine dreidimensionale Abschwächungskorrekturkarte (attenuation correction, AC) des ausgewählten Sichtsfelds und mindestens entweder eine dreidimensionale (3D) Gefäßbilddarstellung oder mindestens ein planares Gefäßangiogramm anhand der empfangenen CT-Projektionsdaten erzeugt;
einen Nuklearbildgebungsscanner (40a, 40b), der Nuklearprojektionsdaten aus der Untersuchungsregion (18) erfasst;
einen Nuklearrekonstruktionsprozessor (44), der programmiert ist, um die erfassten Nuklearprojektionsdaten auf der Grundlage der erzeugten dreidimensionalen Abschwächungskorrekturkarte zu korrigieren und um eine Nuklearbilddarstellung des ausgewählten Sichtfelds anhand der erfassten Nuklearprojektionsdaten auf der Grundlage der korrigierten Nuklearprojektionsdaten zu erzeugen;
**dadurch gekennzeichnet, dass** der CT-Rekonstruktionsprozessor (34) weiterhin konfiguriert ist, um die kontrastverstärkten Gefäße in der dreidimensionalen Volumendarstellung zu segmentieren, die segmentierten kontrastverstärkten Gefäße durch Hintergrundintensitätsdaten zu ersetzen und die dreidimensionale Abschwächungskorrekturkarte auf der Grundlage der CT-Projektionsdaten zu erzeugen, in denen die kontrastverstärkten Gefäße subtrahiert und durch Hintergrundintensitätsdaten ersetzt wurden.

10. Diagnostisches Bildgebungssystem (10) nach Anspruch 9, wobei der CT-Rekonstruktionsprozessor (34) programmiert ist zum:
Filtern der empfangenen CT-Projektionsdaten, um in dem ausgewählten Sichtfeld die Gefäße zu verstärken und die Hintergrundinformationen zu entfernen; und
Rekonstruieren einer dreidimensionalen Bilddarstellung des Sichtfelds anhand der gefilterten Projektionsdaten.

11. Diagnostisches Bildgebungssystem (10) nach Anspruch 10, wobei der CT-Rekonstruktionsprozessor (34) weiterhin programmiert ist zum:
Korrigieren von mindestens entweder den gefilterten Projektionsdaten oder der rekonstruierten dreidimensionalen Bilddarstellung in Hinblick auf Bewegung.

12. Diagnostisches Bildgebungssystem (10) nach einem der Ansprüche 10 oder 11, wobei das Rekonstruieren mit einem iterativen Rekonstruktionsalgorithmus mit mindestens entweder einem Regelungsfaktor, einem Redundanzgewichtungsfaktor oder einem kleinen Aktualisierungsschritt durchgeführt wird.

13. Diagnostisches Bildgebungssystem (10) nach einem der Ansprüche 9 bis 12, wobei der CT-Rekonstruktionsprozessor (34) programmiert ist zum:
Filtern der empfangenen Projektionsdaten, um die Gefäße in dem ausgewählten Sichtfeld zu verstärken;
Erzeugen eines ersten zweidimensionalen trunkierten Angiogramms und eines zweiten zweidimensionalen trunkierten Angiogramms für jeden Projektionswinkel, wobei das zweite zweidimensionale trunkierte Angiogramm einen Projektionswinkel von ungefähr 180° gegenüber dem gegebenen Projektionswinkel während eines ähnlichen Herzbewegungszustands hat; und
Erzeugen eines zweidimensionalen zusammengesetzten Angiogramms für jeden Projektionswinkel durch Verschmelzen des ersten und des zweiten zweidimensionalen trunkierten Angiogramms.

14. Diagnostisches Bildgebungssystem (10) nach Anspruch 9, das weiterhin Folgendes umfasst:
einen Verschmelzungsprozessor (54), der die Nuklearbilddarstellung, die dreidimensionale Gefäßbilddarstellung und das mindestens eine planare Gefäßangiogramms zu einem zusammengesetztem Bild kombinieren; und
eine graphische Benutzeroberfläche (52), die die Nuklearbilddarstellung, die dreidimensionalen Gefäßbilddarstellung, das mindestens eine planare Gefäßangiogramm, das zusammengesetzte Bild oder eine beliebige Kombination hiervon anzeigt.

15. Diagnostisches Bildgebungssystem (10) nach Anspruch 9, das weiterhin Folgendes umfasst:
eine EKG-Vorrichtung (50), die während der Erfassung der CT-Projektionsdaten und der Nuklearprojektionsdaten Elektrokardiogrammdaten (ECG) erfasst; und
Gating der CT- und Nuklearprojektionsdaten entsprechend einem ausgewählten Herzbewegungszustand, bevor die Bilddarstellungen und das mindestens eine Angiogramm erzeugt werden.

## Revendications

1. Procédé d'imagerie de diagnostic comprenant :
la réception de données de projection de tomodensitométrie à augmentation de contraste recueillies à partir d'une région d'examen (18) avec un détecteur à panneau plat latéralement décalé (30) ;
la sélection d'un champ de vision (FOV) qui comprend un ou plusieurs vaisseaux à augmentation de contraste ;
à partir des données de projection de tomodensitométrie reçues, la génération d'une cartographie de correction d'atténuation (AC) 3D du champ de vision sélectionné et au un élément parmi une représentation d'image tridimensionnelle (3D) du vaisseau et au moins un angiogramme plan du vaisseau ;
la réception de données de projection nucléaire recueillies à partir de la région d'examen (18) ;
la correction des données de projection nucléaire recueillies sur base de la cartographie de correction d'atténuation 3D générée ; et
la génération d'une représentation d'image nucléaire du champ de vision sélectionné à partir des données de projection nucléaire recueillies sur base des données de projection nucléaires corrigées ;
**caractérisé en ce que** l'étape consistant à générer la cartographie de correction d'atténuation 3D comprend : la segmentation des vaisseaux à augmentation de contraste dans la représentation de volume 3D, le remplacement des vaisseaux à augmentation de contraste segmentés par des données d'intensité d'arrière-plan, et la génération de la cartographie de correction d'atténuation 3D sur base des données de projection de tomodensitométrie dans lesquelles les vaisseaux à augmentation de contraste sont soustraits et remplacés par les données d'intensité d'arrière-plan.

2. Procédé selon la revendication 1, dans lequel l'étape consistant à générer une représentation d'image tridimensionnelle (3D) de vaisseau, comprend :
le filtrage des données de projection de tomodensitométrie reçues pour accentuer les vaisseaux et pour éliminer les informations d'arrière-plan dans le champ de vision sélectionné ; et
la reconstruction d'une représentation d'image 3D du champ de vision à partir des données de projection filtrées.

3. Procédé selon la revendication 2, dans lequel l'étape consistant à générer une représentation d'image tridimensionnelle (3D) de vaisseau, comprend en outre :
la correction d'au moins un élément parmi les données de projection filtrées et la représentation d'image 3D reconstruite pour le mouvement.

4. Procédé selon l'une quelconque des revendications 1 à 3, dans lequel la reconstruction est effectuée avec un algorithme de reconstruction itératif avec au moins l'un parmi un facteur de régularisation, un facteur de pondération de redondance, et une petite étape de mise à jour.

5. Procédé selon l'une quelconque des revendications 1 à 4, dans laquelle l'étape consistant à générer au moins un angiogramme plan de vaisseau comprend :
le filtrage des données de projection reçues pour accentuer les vaisseaux dans le champ de vision sélectionné ;
pour chaque angle de projection, la génération d'un premier angiogramme 2D tronqué et d'un second angiogramme 2D tronqué, le second angiogramme 2D tronqué possédant un angle de projection approximativement opposé à 180° par rapport à l'angle de projection donné durant un état de mouvement cardiaque similaire ; et
la génération d'un angiogramme 2D composite pour chaque angle de projection en fusionnant les premier et second angiogrammes 2D tronqués.

6. Procédé selon la revendication 1, comprenant en outre :
la combinaison de la représentation d'image nucléaire, de la représentation d'image 3D de vaisseau et de l'au moins un angiogramme plan de vaisseau en une image composite ; et
l'affichage de la représentation d'image nucléaire, de la représentation d'image 3D de vaisseau, de l'au moins un angiogramme plan de vaisseau et de l'image composite.

7. Procédé selon la revendication 1, comprenant en outre :
la réception de données d'électrocardiogramme (ECG) recueillies durant le recueil des données de projection de tomodensitométrie et des données de projection nucléaire ; et
avant de générer les représentations d'image et l'au moins un angiogramme, le portillonnage des données de tomodensitométrie et de projection nucléaire selon un état de mouvement cardiaque sélectionné.

8. Support lisible par ordinateur renfermant un logiciel pour commander un ou plusieurs processeurs pour exécuter le procédé selon l'une quelconque des revendications 1 à 7.

9. Système (10) pour l'imagerie de diagnostic, comprenant :
un scanner à rayons X (24, 30) qui recueille des données de projection de tomodensitométrie à augmentation de contraste provenant d'une région d'examen (18) avec un détecteur à panneau plat latéralement décalé (30) ;
une interface graphique utilisateur (50) pour sélectionner un champ de vision (FOV) qui comprend un ou plusieurs vaisseaux à augmentation de contraste ;
un processeur de reconstruction de tomodensitométrie (34) qui génère une cartographie de correction d'atténuation (AC) 3D du champ de vision sélectionné et au moins un élément parmi une représentation d'image tridimensionnelle (3D) de vaisseau et au moins un angiogramme plan de vaisseau, à partir des données de projection de tomodensitométrie recueillies ;
un scanner d'imagerie nucléaire (40a, 40b) qui recueille des données de projection nucléaire provenant de la région d'examen (18) ;
un processeur de reconstruction nucléaire (44) programmé pour corriger les données de projection nucléaire recueillies sur base de la cartographie de correction d'atténuation générée, et pour générer une représentation d'image nucléaire du champ de vision sélectionné à partir des données de projection nucléaire recueillies sur base des données de projection nucléaires corrigées ;
**caractérisé en ce que** le processeur de reconstruction de tomodensitométrie (34) est en outre configuré pour segmenter les vaisseaux à augmentation de contraste dans la représentation de volume 3D, pour remplacer les vaisseaux à augmentation de contraste segmentés par des données d'intensité d'arrière-plan, et pour générer la cartographie de correction d'atténuation 3D sur base des données de projection de tomodensitométrie dans lesquelles les vaisseaux à augmentation de contraste ont été soustraits et remplacés par des données d'intensité d'arrière-plan.

10. Système d'imagerie de diagnostic (10) selon la revendication 9, dans lequel le processeur de reconstruction de tomodensitométrie (34) est programmé pour :
filtrer les données de projection de tomodensitométrie recueillies pour accentuer les vaisseaux à augmentation de contraste et pour éliminer les informations d'arrière-plan dans le champ de vision sélectionné ; et
reconstruire la représentation d'image 3D de vaisseau du champ de vision à partir des données de projection filtrées.

11. Système d'imagerie de diagnostic (10) selon la revendication 10, dans lequel le processeur de reconstruction de tomodensitométrie (34) est en outre programmé pour :
corriger au moins un élément parmi les données de projection filtrées et la représentation d'image 3D reconstruite de vaisseau pour le mouvement.

12. Système d'imagerie de diagnostic (10) selon l'une quelconque des revendications 10 et 11, dans lequel la reconstruction est effectuée avec un algorithme de reconstruction itératif avec au moins l'un parmi un facteur de régularisation, un facteur de pondération de redondance, et une petite étape de mise à jour.

13. Système d'imagerie de diagnostic (10) selon l'une quelconque des revendications 9 à 12, dans lequel le processeur de reconstruction de tomodensitométrie (34) est programmé pour :
filtrer les données de projection recueillies pour accentuer les vaisseaux dans le champ de vision sélectionné ;
générer un premier angiogramme 2D tronqué et un second angiogramme 2D tronqué pour chaque angle de projection, le second angiogramme 2D tronqué possédant un angle de projection approximativement opposé à 180° par rapport à l'angle de projection donné durant un état de mouvement cardiaque similaire ; et
générer un angiogramme 2D composite pour chaque angle de projection en fusionnant les premier et second angiogrammes 2D tronqués.

14. Système d'imagerie de diagnostic (10) selon la revendication 9, comprenant en outre :
un processeur de fusion (54) qui combine la représentation d'image nucléaire, la représentation d'image 3D de vaisseau et l'au moins un angiogramme plan de vaisseau en une image composite ; et
une interface graphique utilisateur (52) qui affiche la représentation d'image nucléaire, la représentation d'image 3D de vaisseau, l'au moins un angiogramme plan de vaisseau, l'image composite ou n'importe quelle combinaison de ceux-ci.

15. Système d'imagerie de diagnostic (10) selon la revendication 9, comprenant en outre :
un dispositif ECG (50) qui recueille des données d'électrocardiogramme (ECG) durant le recueil des données de projection de tomodensitométrie et des données de projection nucléaire ; et
avant de générer les représentations d'image et l'au moins un angiogramme, le portillonnage des données de tomodensitométrie et de projection nucléaire selon un état de mouvement cardiaque sélectionné.
